# EUROPEAN PATENT SPECIFICATION

(11) **EP 4 241 845 B1**
(45) Date of publication and mention of the grant of the patent: **10.07.2024**
(21) Application number: 22382207.3
(22) Date of filing: 07.03.2022
(51) Int. Cl.: A61P 31/22, A61K 31/695, A61K 9/00, A61K 47/24, A61K 36/52, A61K 31/122, A61K 9/06, A61K 47/34

(54) **SILICONE GEL COMPOSITION OF WALNUT EXTRACT**
SILIKONGELZUSAMMENSETZUNG AUS WALNUSSEXTRAKT
COMPOSITION DE GEL DE SILICONE À BASE D'EXTRAIT DE NOIX

(43) Date of publication of application: 13.09.2023
(73) Proprietor: Hartington Business, S. L., 08302 Mataro (ES)
(72) Inventor: MTCHEDLIDZE KVIRKVELIA, Vakhtang, Barcelona (ES)
(74) Representative: Ponti & Partners, S.L.P

(56) References cited:
- US-A1- 2010 080 768
- MATSUMOTO M ET AL: "Selective nonpeptidic inhibitors of herpes simplex virus type 1 and human cytomegalovirus proteases", BIOLOGICAL & PHARMACEUTICAL BULLETIN, PHARMACEUTICAL SOCIETY OF JAPAN, TOKYO, JP, vol. 24, no. 3, 1 January 2001 (2001-01-01), pages 236 - 241, XP002472659, ISSN: 0918-6158, DOI: 10.1248/BPB.24.236
- BINDER R G ET AL: "Eight 1,4-naphthoquinones from Juglans", PHYTOCHEMISTRY, ELSEVIER, AMSTERDAM , NL, vol. 28, no. 10, 1 January 1989 (1989-01-01), pages 2799 - 2801, XP026633610, ISSN: 0031-9422, [retrieved on 19890101], DOI: 10.1016/S0031-9422(00)98092-0
- ANONYMOUS: "Liveo(TM) ST-Elastomer 10", 11 September 2020 (2020-09-11), pages 1 - 3, XP055959336, Retrieved from the Internet <URL:http://www.healthcare-plus.com.tw/big5/pdf/01-07.pdf> [retrieved on 20220909]
- DATABASE GNPD [online] MINTEL; 21 May 2009 (2009-05-21), ANONYMOUS: "Voile Eclat d'Été Tan Booster", XP055959646, retrieved from https://www.gnpd.com/sinatra/recordpage/1105127/ Database accession no. 1105127

## Description

### Field of the invention

The present invention is in the field of topical emollient gel compositions, which contains silicones and natural extract of walnut fruits suitable for preparing an anti-herpes viruses product.

The present invention also relates to the emollient gel composition for topical use in the treatment of herpes viruses at a posology regime.

### Background of the invention

Recently, in light of herpes viruses treatment, there has been a demand for the development of natural preparations with improved efficacy.

However, the therapeutic effect of external preparations is greatly influenced by each drug's ability to permeate the skin.

Silicones are well known in the art for external preparations. Silicones are highly diverse, and mainly are using for cosmetic formulations. In addition to being vegan-friendly, hypoallergenic, colorless, and odorless, they also have sensory properties.

However, the low penetration of silicones when applied topically over human skin is well known in the art, see for example, M. B. Reddy, R. J. Looney, M. J. Utell, K. P. Plotzke, M. E. Andersen in "Modeling of Human Dermal Absorption of Octamethylcyclotetrasiloxane (D4) and Decamethylcyclopentasiloxane (D5)", Toxicological Sciences, 2007; 99 (2), 422-431.

On the one hand, US 9,511,034 directed to a "Method for applying a skin treatment" discloses a skin treatment for managing skin conditions ranging from scars to burns and wrinkles. The skin treatment may comprise several components or layers: a cleansing solution component, a silicone elastomer component, and a mineral powder component. In exemplary embodiments, three layers or components have been used to treat and camouflage a scar: a solution for cleansing the skin, a silicone elastomer for treating the cleansed skin, and a mineral powder for further protecting and camouflaging the treated skin area. A long list of additional additives are mentioned including walnut extract (Juglans nigra). However, the walnut extract is as a source of brown coloring.

EP 2,811,986 directed to "Silicone emulsions for delivery of healthcare actives" discloses a composition comprising: i) an aqueous silicone emulsion comprising; A) 0.5 wt. % to 95 wt. % of a silicone gum, resin, or pressure sensitive adhesive (PSA), B) 0.1 to 90 wt. % of an ethylene oxide/propylene oxide block copolymer, and sufficient amount of water to sum all ingredients of the silicone gum emulsion to 100 weight percent, ii) a healthcare active, and iii) an optional enhancer(s). Certain silicone gum, resin, or pressure sensitive adhesive (PSA) emulsions are described for preparing dermal formulations to deliver healthcare actives. The actives can be a protein, such as an enzyme. The active may also be one or more plant extract from a long list including walnut extract. However, these compositions have been formulated for treating fungal infections, not viruses, and their objectives are to facilitate an enhanced rate of penetration for effectiveness of these actives.

In a similar way, EP 2,385,819 describes silicone paste compositions. EP 2,385,819 deals with the problem of compositions enough compatibilizing to be stable when formulated as a personal or healthcare product. The compositions combines a silicone organic elastomer gel with water or a hydrophilic solvent that may further contain a personal or healthcare active. However, EP 2,385,819 is in the field of fungal infections, not viruses.

On the other hand, Juglone, also called 5-hydroxy-1,4-naphthalenedione (IUPAC), is an organic compound with the molecular formula C₁₀H₆O₃. Juglone can be found naturally in the walnut tree, which contains at the bark, leaves or fruits gallic and catechin tannins, juglone, juglandine, carotene, inositol, pyrogallol, vitamin C and other substances. The anti-microbial, anti-viral and anti-bacterial properties of natural Juglone are known, for example, from EP 2,040,727 B1 of the same applicant.

US 6,296,838 refers to a topical composition including a Juglone extract. In particular, it discloses that the extract having synergic effects should contain a mixture of 10-15% of Juglone extract from walnut hull with 20-30% of milled roots of Nardostachys jatamansi o Vetiveria zizanioides o Catharanthus roseus, because an extract of only Juglone showed very little efficiency even in the long curing treatment and fungi-toxic effect. US 6,296,838 is in the field of treating and preventing human nails infected with fungi. Not suggestion or disclose is made for viruses.

Therefore, there is not yet in the art, external preparations for topically treating herpes viruses with enhanced effectiveness.

In fact, the state of the art does not deal with the problem of effectiveness vs skin permeation of a topical composition, but focused on the rate of penetration for active effectiveness as discussed above in EP 2,811,986.

Thus, it is still desirable to provide a natural composition capable of active permeating at the target site of the skin where viruses stand for improving effectiveness and reducing the curing period. To achieve this goal may result in an improved effectiveness of the treatment.

Moreover, retention of the composition for a certain period in the target site of skin is also required for effectiveness and alleviative effect of the silicone topical composition.

Thus, it is also still desirable to provide a silicone composition for topical use in the symptomatic relief of herpes viruses with high effectiveness.

It is also desirable to provide a natural silicone composition stable in storage, and particularly excellent stability notwithstanding the known low stability of the natural components contained in the walnut extract.

### Brief description of the invention

The present invention was made in view of the prior art described above. The present invention provides a silicone gel composition comprising walnut extract for preparing an anti-herpes viruses product with improved effectiveness. In particular, the invention relates to an anti-herpes viruses topical gel composition comprising a combination of three types of silicones capable of permeating walnut extract at a target site of the skin in a depth range to deliver natural naphthoquinones therein. Silicone gel composition of the invention is capable of permeating at the target site of epidermis.

The silicone gel composition includes a natural extract of unripe walnut fruit (*Juglans Regia L*.) including natural naphthoquinones ingredient.

Advantageously, the composition of the first aspect provides a good permeation of the walnut extract components in the skin. In particular, the silicone gel composition of the invention provides a good natural naphthoquinones ingredient permeation at the target site of epidermis, thereby conferring systemic therapeutic safety because of it does not absorb nor penetrate at the dermis site.

The authors of the invention have found that the silicone gel composition of the first aspect does not absorb, not penetrate at the dermis depth, but is capable of permeating at the target site of the epidermis depth range.

Advantageously, the silicone gel composition of the invention possesses permeation at the epidermal zone in a depth range of about 30 to about 120 µm.

Additionally, the silicone gel composition of the first aspect shows improved retention of the composition at the target site of the epidermis zone and that retention is for a period, thus still further improving effectiveness.

Surprisingly, a concentration between 500 and 800 ng/cm² of natural naphthoquinone ingredient (in particular, juglone) has been found for at least 16 hours in the epidermal depth range, after topical application of the silicone gel composition of the invention in the posology regime defined in the second aspect of the invention.

Thus, the second aspect of the present invention provides the silicone gel composition of the first aspect for topical use in the treatment of herpes viruses at a dosage regime, administered at 3-5-hourly intervals, of 125 to 175 mg up to a total amount of 500 to 600 mg.

### Brief Description of the Drawings

**Figure 1** shows the human skin layers and the range of thickness of each of the layers. The epidermal layer is in the range of 30 to 120 µm. The silicone gel composition of the invention aimed at treating the epidermal zone.
**Figure 2.1** shows calibration line of juglone standard solution between 100 ng/mL and 5000 ng/mL in methanol/water 80:20 v/v, and **Figure 2.2** shows the spectral information of the presence of juglone standard 5 ppm obtained by HPLC MS/MS.
**Figure 3** shows the spectral information obtained by HPLC MS/MS of permeation of natural naphthoquinone ingredient (in particular, juglone) through human skin to collector solution after treatment with 600 mg of the silicone gel composition of the invention.
**Figure 4** shows the spectral information obtained by HPLC MS/MS of natural naphthoquinone ingredient (in particular, juglone) retained in human skin to extraction of natural naphthoquinone ingredient (in particular, juglone) from 2.54 cm² skin (400 µm thick) treated with 600 mg of the silicone gel composition of the invention.

### Detailed description of the invention

Thus, the first aspect of the invention provides a silicone gel composition comprising walnut extract suitable for topical treatment of herpes family viruses and capable of permeating natural naphthoquinone ingredient at the target site of the epidermis zone.

The silicone gel composition comprising walnut extract is characterized in that the composition comprises:
- a silicone mixture consisting of: a silicone elastomer gel, a silicone fluid base, and a silicone emulsifier; and
- a walnut extract including natural naphthoquinones ingredient,

wherein the silicone elastomer gel is in an amount between 60 and 75%, the silicone fluid base is in an amount between 12 and 25%, the silicone emulsifier is in an amount between 2 and 6%, and the walnut extract is in an amount between 5 and15%, and
wherein the percentages are expressed by weight with respect to the weight of composition, provided that the sum of all components does not exceed 100%.

Advantageously, the silicone gel composition of the invention is capable of permeating at the target site of the epidermis zone.

The present invention additionally relates to a silicone gel composition capable of retaining a walnut extract concentration at the target site of the epidermis zone for a period. Therefore, while ensuring retention of walnut extract at the target site of epidermis, further improved efficacy of the composition.

In an embodiment, the silicone gel composition of the invention may further optionally includes one or more topical excipients.

The silicone gel composition has an appearance of a semisolid gel.

The selection of silicone elastomer gel, silicone fluid base, and silicone emulsifier in the ranges described herein combined with the walnut extract including natural ingredient provides the advantages herein described.

Advantageously, the silicone gel composition of the invention reveal low or no penetrability in the dermis above the vascular plexus, which confers systemic therapeutic safety.

Moreover, the silicone gel composition of the invention has excellent storage stability.

Preferable silicone elastomer gel includes decamethylcyclopentasiloxane (D5) in a network backbone of polydimethylsiloxane (PDMS), also known as cyclomethicone dimethicone crosspolymer.

In an embodiment, the silicone elastomer gel includes decamethylcyclopentasiloxane (D5) in an amount of about 85% by weight and a crosslinked polymer of average molecular weight higher than 5,000 in an amount of about 12% by weight.

Preferable silicone fluid base includes a cyclopentasiloxane compound.

In an embodiment, the cyclopentasiloxane compound includes decamethylcyclopentasiloxane (D5) in an amount higher than about 95% by weight.

Preferable silicone emulsifier includes alkylmethyl silicone polyether copolymer.

As described above, the silicone mixture consists of a mixture of a silicone elastomer gel, a silicone fluid base, and a silicone emulsifier. Components of the silicone mixture are detailed below.

### - Silicone Elastomer Gel-

The silicone elastomer gel is an apolar material, incompatible with hydrophilic materials.

Its physical form at 25°C is a paste (gel). Its color is crystal clear to slightly translucent. May have slight yellow or brownish color.

As described above, preferable silicone elastomer gel includes decamethylcyclopentasiloxane (D5) in a network backbone of polydimethylsiloxane (PDMS). In the network, the polydimethylsiloxane chains are cross-linked by shorter polydimethylsiloxane chains and hexyl chains. The network is swollen in the presence of decamethylcyclopentasiloxane (D5).

In an embodiment, silicone elastomer gel includes a high molecular weight crosslinked polymer (about 12% w.) in decamethylcyclopentasiloxane (D5) (about 85% w.). The crosslinked polymer of high molecular weight includes a crosslinked polymer with an average molecular weight higher than 5,000.

Commercially available in the market can be found as "ST-Elastomer-10 ^{®}" sold by Down Corning Corporation, having a viscosity between 350,000 and 490,000 cSt (Centistokes [cSt] = 1 mm²/s) (also named cyclomethicone dimethicone crosspolymer). The high molecular weight silicone elastomer (around 12%) and the decamethylcyclopentasiloxane (D5) (around 85%) are the major components. The impurities are octamethylcyclotetrasiloxane and others cyclomethylsiloxanes, and residual short vinyl polymer (dimethylvinylsiloxy-terminated dimetylsiloxane). "ST-Elastomer-10 ^{®}" is a silicone elastomer particles dispersed and swollen in a volatile silicone solvent, decamethylcyclopentasiloxane (also known as cyclomethicone 5 or D5). The elastomeric silicone particles are made of a crosslinked polymer of an aMW > 5,000, obtained by the hydrosilylation reaction between SiH groups of a high MW SiH silicone polymer (trimethylsiloxy-terminated dimethyl methylhydrogen siloxane), and the vinyl group of 1,5-hexadiene in the presence of platinum catalyst and decamethylcyclopentasiloxane.

The silicone elastomer gel (after the hydrosilylation reaction leading to the elastomeric network and formation of the gel) has a composition including: decamethylcyclopentasiloxane (D5) (about 85% w.), reaction product of dimethyl methylhydrogen silixane with 1,5-hexadiene (about 12% w.); octamethylcyclotetrasiloxane (D4) (maximum 0.9% w.); dimethyl siloxane, dimethylvinylsiloxy-terminated (about 0.6 % w.); and dimethylcyclosiloxanes (1% w.).

Preferably, the silicone elastomer gel can be present in the mixture in an amount from 65 to 72%, more preferably between 67 and 69%, still more preferably about 68% by weight with respect to the weight of composition of the first aspect of the invention.

### - Silicone Fluid Base -

The silicone fluid base is a volatile fluid with appreciable vapor pressure at ambient temperature.

As described above, preferable silicone fluid base includes a cyclopentasiloxane compound. The cyclopentasiloxane is a polydimethylcyclopentasiloxane composed mainly of decamethylcyclopentasiloxane (>95%).

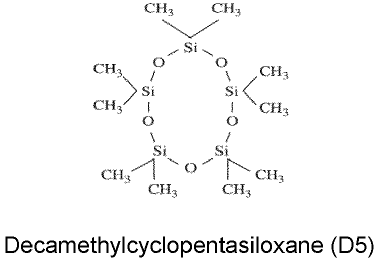

In an embodiment, the silicone fluid base includes decamethylcyclopentasiloxane (D5) in an amount higher than 95% by weight of the weight of silicone fluid base component.

Commercially available in the market can be found as "ST-Cyclomethicone 5 NF^{®}" sold by Down Corning Corporation, having a viscosity at 25°C of ≈ 3.8 mPa/s (4.0 mm².s⁻¹). It may be used alone or blended with other silicones or organic excipients to provide a fluid base for a variety of formulations.

Preferably, the silicone fluid base can be present in the silicone mixture in an amount between 17 and 19%, more preferably between 14 and 20%, still more preferably about 18% by weight with respect to the weight of composition of the first aspect of the invention.

### - Silicone Emulsifier-

The silicone emulsifier is a liquid silicone that enables the formulation of creams and lotions at room temperature. The silicone emulsifier component in the composition of the first aspect allows forming a very stable visco-elastic film once topically composition is applied on human skin. The film forming protects and prevents colonization by viruses of the herpes virus family, and achieves transdermal permeation of the naphthoquinones contained in the walnut extract in the epidermis depth range, while not reaching the dermis and thus achieving safe use on skin affected by viral infections.

As described above, preferable silicone emulsifier includes a (liquid) alkylmethyl silicone polyether copolymer. The silicone emulsifier leads to the silicone gel composition excellent stability at high water concentration and low emulsifier levels.

Commercially available in the market can be found as "Dowsil-5200 Formulation Aid^{®}", Lauryl PEG/PPG - 18/18 Methicone, sold by Down Corning Corporation, having a viscosity at 25°C between 1,100 to 2,600 cSt (Centistokes [cSt] = 1 mm²/s) and a flash point of 92.5°C, a low hydrophilic-to-lipophilic balance. Dowsil is effective at producing water-oil emulsions with a low to medium polarity oil phase or water-in-silicone emulsion, and can be used alone or blended with other silicones or organic excipients to provide a fluid base for a variety of formulations. Lauryl methicone PEG/PPG-18/18 is an alkoxylated derivative of lauryl methicone containing an average of 18 moles of ethylene oxide and 18 moles of propylene oxide.

Preferably, the alkylmethyl silicone polyether copolymer component can be present in the silicone mixture in an amount between 2 and 6%, preferably between 3 and 5%, more preferably about 4% by weight with respect to the weight of composition of the first aspect of the invention.

### - Walnut extract including natural naphthoquinones ingredient-

The silicone gel composition of the first aspect of the invention further comprises walnut extract in an amount between 5 and 15% by weight with respect to the weight of composition.

As known in the art walnut extract (*Juglans Regia L.*) includes natural naphthoquinones (in particular, juglone), usually in an amount between 0.1 and 0.5% by weight.

As disclosed above, the walnut extract is present in an amount between 5 and 15%, preferably between 8 and 12%, more preferably about 10% by weight with respect to the weight of composition of the first aspect of the invention.

Walnut extract can be obtained from any extraction method known in the art. However, preferable walnut extract is obtainable following the method described in the granted European Patent No. EP 2,040,727-B1.

The general method of EP 2,040,727 includes the following steps:
i) Collecting unripe walnut fruits as raw material; ii) Preparing the raw material for the extraction; iii) Freezing the raw material prepared in the previous step; iv) Drying; v) Extracting in a time lower than 10 minutes, wherein the extraction step (v) comprises breaking the dried mass obtained in the drying step into pieces and adding an alcohol in a 1/2 to 1/5 weight/volume ratio and carrying out an extraction during a time lower than 10 minutes; and; vi) Filtration.

In the freezing step (iii) it is preferable keeping the walnuts in a frozen chamber at a temperature comprised between -18 and -20°C during 70-170 hours. And in the drying step (iv) it is preferable first peeling the shells of the frozen walnut in thin layers of 2 to 3 mm, and then drying until a humidity lower than 10% is obtained.

In an embodiment, the silicone gel composition comprises:
- a silicone mixture consisting of:
   60-75% of cyclomethicone dimethicone crosspolymer,
   10-25 % of cyclopentasiloxane, and
   2-6% of alkylmethyl silicone polyether copolymer,
      and
- a walnut extract in an amount between 5 and 15%,
wherein the percentages are expressed by weight with respect to the weight of composition, provided that the sum of all components does not exceed 100%.

In another embodiment, the silicone gel composition comprises:
- a silicone mixture consisting of:
   67-69% of cyclomethicone dimethicone crosspolymer,
   17-19 % of cyclopentasiloxane,
   3-5% of alkylmethyl silicone polyether copolymer,
      and
- a walnut extract in an amount between 9 and 11%,
wherein the percentages are expressed by weight with respect to the weight of composition, provided that the sum of all components does not exceed 100%.

In a more preferable embodiment, the silicone gel composition comprises:
- a silicone mixture consisting of:
   68% cyclomethicone dimethicone crosspolymer as the silicone elastomer gel;
   18% cyclopentasiloxane compound as the silicone fluid base, and
   4% alkylmethyl silicone polyether copolymer as the silicone emulsifier,
      and
- a walnut extract in an amount about 10%,
wherein the percentages are expressed by weight with respect to the weight of composition, provided that the sum of all components does not exceed 100%.

In still another preferable embodiment, the silicone gel composition comprises 10% walnut extract; 68% cyclomethicone and dimethicone crosspolymer as elastomer; 18% cyclopentasiloxane compound as the base, and 4% alkylmethyl silicone polyether copolymer as emulsifier, wherein the percentages are expressed by weight of the total weight of the composition and the sum of all components does not exceed 100%.

The silicone gel composition of the first aspect has a semisolid texture that facilitates the topically application *versus* a liquid texture. In an embodiment, the semisolid texture is a semisolid gel.

In a second aspect, the present invention relates to the topical use of the silicone gel composition of the first aspect of the invention for the treatment of viruses of the herpes family at a posology regime.

The silicone gel composition as defined in the first aspect is suitable for topical use in the treatment of herpes virus.

The use comprises a dosage regime, administered at an interval of 3 to 5 hours, of 125 to 175 mg up to a total amount in the range of 500 to 600 mg.

In an embodiment, the dosage regime, administered at 4-hourly intervals, is of 125 to 175 mg up to a total of 500 mg.

In another embodiment, the dosage regime, administered at 4-hourly intervals, is of 150 mg up to a total of 600 mg.

Advantageously, the silicone gel composition of the first and second aspects of the invention possesses naphthoquinone (in particular, juglone) permeation at the epidermal zone in a depth range of about 30 to about 120 µm.

The topical use of silicone gel composition of the invention shows improved retention of the active ingredient at the target site of the epidermis zone and that retention is for a period, thus still further improving effectiveness.

Surprisingly, at the posology regime described above, naphthoquinone is retained at a concentration between 500 and 800 ng/cm² for at least 16 hours in the epidermal depth range.

### - ASSAYS -

The authors of the present invention have performed several studies to analyze the properties of the silicone gel composition of the first/second aspect of the invention.

A first study was focused on the permeation rate of juglone (natural naphthoquinone) through human skin.

A second study was focused on extraction of the amount of natural naphthoquinone retained in human skin.

The amount retained in the samples generated during the study and the depth reached in the skin have been measured at the laboratory using a liquid chromatography-mass spectrometry analytical method previously qualified and of proven suitability and using the confocal microscopy technique.

### - Confocal optical microscopy -

The penetration in human skin was also studied by confocal microscopy, using the fluorescence of the Juglone molecule exciting it with light of 488 nm and observing its fluorescence in a window from 510 to 702 nm. For this penetration study, a Leica microscope, model SPE Confocal Microscope was used, with a 10 x lens. The location of the Juglone molecules in depth was established with this instrument (in perpendicular direction to the skin surface). The accumulation of Juglone at different depth levels causes the appearance of areas with different fluorescence intensity and allows to observe directly where the molecule is retained. It must be noted that white Caucasian skins were chosen to prevent interferences of basal fluorescence and thus clearly observe the fluorescence of the Juglone molecule.

### - Permeation of Juglone through the human skin -

The transdermal permeation study was performed according to the "Guidance document for the conduct of skin absorption studies". OECD series on testing assessment. Number 28. ENV/JM/MONO (2004) 2.05-Mar-2004. Using Franz type cells, of 1.80 cm in diameter, and a surface of 2.54 cm². The permeation membrane used was human skin of the abdominal area (0.400 mm thick) from repair surgery and previously treated. This thickness covers the epidermis and dermis above the vascular plexus. The integrity of the skin used has been checked by estimation of the value of the parameter TEWL (Transepidermal Water Loss). The study of Juglone retention in the skin was performed considering the epidermal area between surface and 0.150 mm in depth and dermal area above the vascular plexus between 0.150 and 0.400 mm in depth. For each study, human skin from a population sample (n=6) was used so that the results were representative of a group providing inter-individual differences.

In the cell donor compartment 600 mg of silicone gel composition including 10% of walnut extract and a Juglone content in the silicone gel composition of 0.04% w/w were applied in four applications at 0, 4, 8 and 14 hours, of 150 mg each, collecting the receiving fluid at 16 hours. It was applied rubbing gently over the skin, simulating application with the fingertip. As receiving solution, a deionized water/ethanol solution, 90:10, v/v, was used, which enables to keep the SINK conditions throughout the study; in addition, the cell was maintained at 32 ± 1 °C using a heating jacket. Sampling was performed from the receiving compartment at the following times: 4, 8, 14 and 16 h. The sample volume taken was 0.3 mL.

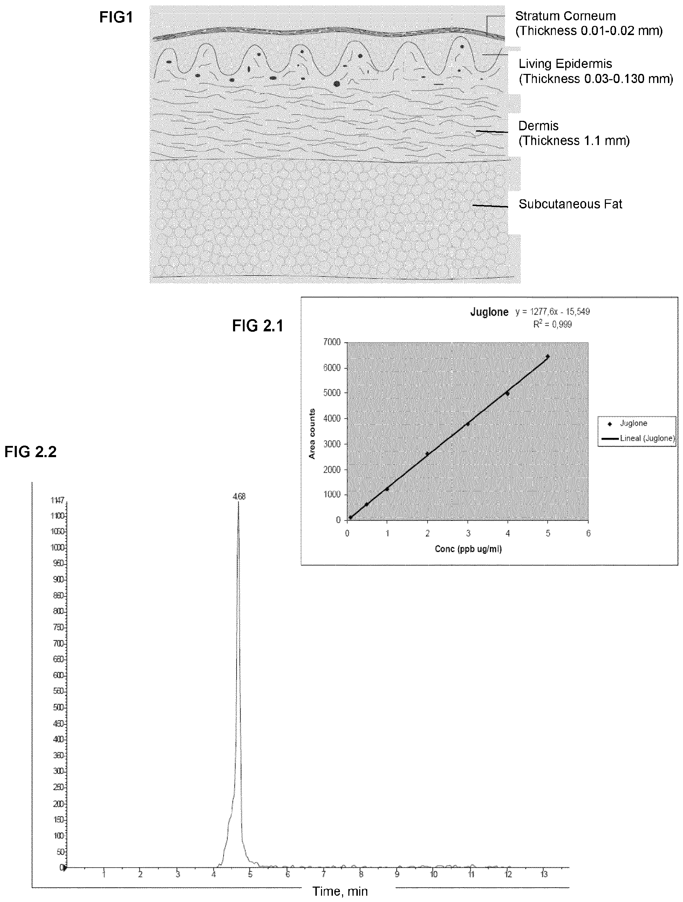

To study the Juglone content that crosses the skin and the amount therein retained, two studies were performed using the silicone gel composition of the invention. In the two studies, the silicone gel composition included 10% of walnut extract (0.04% w/w Juglone). The first with human skin placed in five Franz cells, as described above, with no other actuation; and a second study consisting of sectioning horizontally the skin sample placed in a sixth Franz cell, dividing it into two layers: one of 0.150 mm in thickness (from 0 mm to 0.150 mm in depth (epidermis)) and another of 0.250 mm in thickness (from 0.150 mm to 0.400 mm in depth (dermis over the vascular dermis plexus)). The first peeled layer, on the one hand, and the rest, on the other hand, were sonicated with methanol/water 80:20, v/v, keeping temperature constant at 25°C and protecting it from light to be able to estimate the area were Juglone was mainly fixed in the skin, as the epidermis is not delimited in an exact, defined thickness (Figure 1), but it shows invaginations that make it irregular both in the surface and in depth.

### - Study on extraction of the Juglone retained -

The determination of Juglone retained in human skin was performed after washing the external surface of the skin with a 0.1% sodium lauryl sulphate solution and subsequent washings with HPLC grade water. After this, following skin drying and weighing, the approximate depth of Juglone penetration in it was performed using the confocal microscopy technique.

The next study step was that five of the six skins used as membrane were sank in a methanol/water solution 80:20, v/v. With this Juglone was taken from the portions of skin treated with the silicone gel composition, corresponding to the extraction of Juglone throughout the skin thickness (0.400 mm). The method used includes sonication, for 20 minutes, under bath temperature control (25 °C).

The study on extraction of Juglone retained in human skin was performed immediately after observing under the confocal optical microscope five of the six pieces. The skin disc was placed in glass vials adding to them one milliliter of methanol/water 80:20 v/v, stirred, sonicated for 20 minutes in an ultrasonic bath of 100 w of power, and allowed to stand for half an hour. The extracting fluid was transferred to two vials of automated injector for chromatography for analysis by HPLC-MS/MS.

The sixth skin sample was treated in the same way, but in two portions. The first was the superficial or epidermal, cut from 0 mm to 0.150 mm in depth with a Leica cryomicrotome. The second was the remaining piece, from 0.150 mm to 0.400 in depth, corresponding to the upper dermis area of the vascular plexus. One squared centimeter of each area was used. The corresponding extraction solutions, performed with 500 µL of methanol/water, 80:20, v/v, were transferred to both vials of automated injector for chromatography for analysis by HPLC-MS/MS.

The preparation of the calibration standards and the analytical samples were carried out as follows. The samples from Juglone extraction absorbed in human skin with 1 mL of methanol/water 80:20, v/v, were analyzed with HPLC-MS/MS. A stock solution containing 1.00 mg of Juglone standard (commercial standard Aldrich HA47003), 500 µL of methanol, 400 µL of acetone and 100 µL of deionized water (1000 ppm of Juglone standard in solution) was prepared and filtered through a Teflon membrane of 0.22 µm of pore. From this, solutions of 100, 500, 1000, 2000, 3000, 4000 and 5000 µg/L of Juglone standard (ppb) were prepared to build the calibration line. The samples were taken directly to the automatic injector of the chromatograph with no other preparation (Figure 2). Operating conditions HPLC-MS/MS were the followings: HPLC (Agilent 1100 Series); Mass spectrometer (API3000-AB Sciex); Chromatographic column: Luna 5 µm C18(2) 150X2 mm; Flow rate: 0.80mL/min; Injection volume:10µL; Mobile phase: A) 10mM ammonium acetate pH 6 in water; B) acetonitrile. Elution gradient:

| **Time, min** | **Eluent A, %** | **Eluent B, %** |
|---|---|---|
| 0 | 85 | 15 |
| 0.5 | 85 | 15 |
| 8 | 5 | 95 |
| 10 | 5 | 95 |
| 10.5 | 85 | 15 |
| 15 | 85 | 15 |

### Results

- *Confocal optical microscopy*: The images corresponding to Juglone distribution in depth were generated, observing that the specific fluorescence of Juglone molecule is maintained in the region that can be called epidermal, at less than 120 µm in depth, and the signal disappeared at greater depths.

Table 1 below shows the depth range where fluorescence was seen due to the presence of Juglone, in human abdominal skin sections of 400 µm in thickness. The zero level corresponds to the surface of the epidermis.

**Table 1**

| Donor | Fluorescence observed to a depth of (µm) |
|---|---|
| 1 | 90.82 |
| 2 | 87.89 |
| 3 | 117.19 |
| 4 | 102.54 |
| 5 | 99.61 |
| 6 | 120.117 |

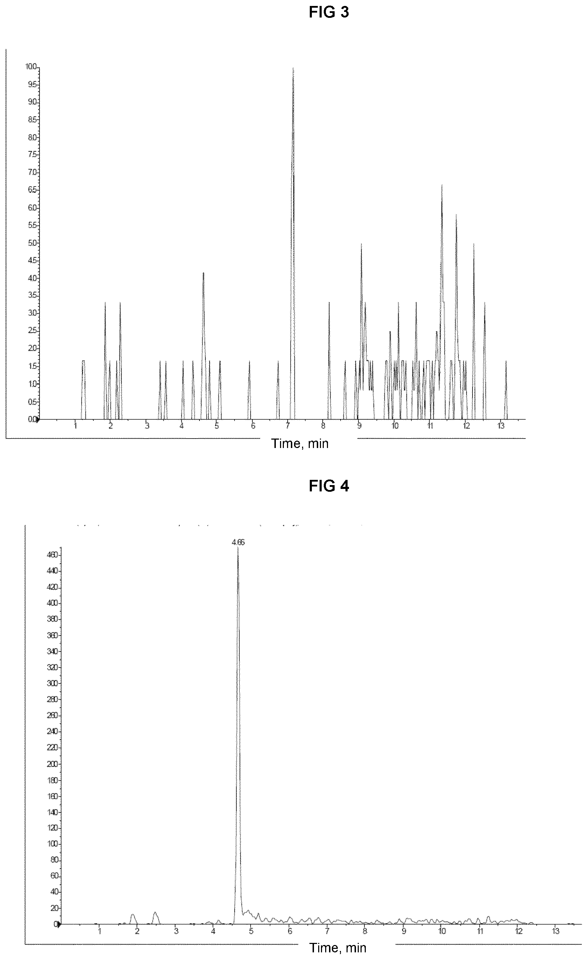

• *HPLC-MS*/*MS, Results of the permeation study*: Table 2 shows the amounts of Juglone permeated through human skin of 400 µm in thickness for each donor (n=6) and for the silicone gel composition, expressed in ng/cm², at each time tested, after four sequential applications of 150 mg each (times = 0, 4, 8 and 14 h, respectively). The value of the median is given as central trend parameter as they are biological samples with a high variability (Figure 3).

**Table 2**

| Time (h) | Donor 1 | Donor 2 | Donor 3 | Donor 4 | Donor 5 | Donor 6 | Average |
|---|---|---|---|---|---|---|---|
| 4 | <180 | <180 | <180 | <180 | <180 | <180 | * |
| 8 | <180 | <180 | <180 | <180 | <180 | <180 | * |
| 14 | <180 | <180 | <180 | <180 | <180 | <180 | * |
| 16 | <180 | <180 | <180 | <180 | <180 | <180 | * |

| | | | | | | | |
|---|---|---|---|---|---|---|---|
| (*) Below the limit of quantitation of the technique: 180 ng of Juglone/cm². | | | | | | | |

• *HPLC-MS*/*MS, Results of the study on retention of Juglone in human skin:* Table 3 shows the amounts of Juglone taken from human skin of 0.400 mm in thickness for the tested silicone gel composition (n=5) and applications, expressed in absolute quantities (ng), for a surface of 2.54 cm² and in ng/cm² (Figure 4).

**Table 3**

| Sample (skin thickness 0.4 mm) | Amount of Juglone taken from 2.54 cm² of skin (ng) | Amount of Juglone taken from 1 cm² of skin (ng/ cm²) |
|---|---|---|
| 2 | 1748 | 688 |
| 3 | 1846 | 727 |
| 4 | 2443 | 962 |
| 5 | 1968 | 775 |
| 6 | 1998 | 799 |

Table 4 below shows, for the silicone gel composition tested after the applications, the amounts of Juglone taken from human skin in two horizontal sections, one the epidermal with a thickness from 0 mm to 0.150 mm (thickness of 0.150 mm) and the other from 0.150 mm to 0.400 mm (thickness 0.250 mm) corresponding to the dermis, expressed in nanograms for a surface of 2.54 cm² treated and in ng/cm², that was retained in the epidermis and dermis above the vascular plexus. It is associated with the ratio of the two quantities, clearly favorable to the epidermis vs the dermis.

**Table 4**

| Sample 1 (skin thickness, mm) | Amount of Juglone taken from 2.54 cm²of skin (ng) | Amount of Juglone taken from 1 cm²of skin (ng/cm²) | Distribution of Juglone epidermis/dermis |
|---|---|---|---|
| **0.150** (0-0.150 mm in depth) | 1958 | 771 | 3.74 |
| **0.250** (0.150-0.400 mm in depth) | 523 | 206 | 1 |

### Conclusions

The transdermal permeation study evidence that the amount of Juglone permeated through human skin at 16 hours of the study (by accumulation). Given these results all skins show a similar systemic safety profile.

Concerning the product amounts retained in human skin, susceptible of having some effect "in situ", significant differences were found between epidermis and dermis in the study. As silicone gel composition including 10% wt. of walnut extract and a Juglone content of 0.04% w/w remained 16 hours on the skin, after administration of a total dose of 600 mg of silicone gel composition (four applications of 150 mg each), the analytical techniques used allowed to find 3.74 times more Juglone in the epidermal than in the dermal zone.

The amounts of Juglone taken from five skins from different donors evidence that this silicone gel composition promotes the stay of Juglone at the site of application, which may be translated into a higher efficacy. The microscopy tests support the observation made by analytical-chemical route.

The amount of Juglone permeated after applying multiple doses through the human skin was not detected at 16 hours of the test, so it can be stated that it is below the limit of quantitation of the technique (180 ng/cm² in this study) at 16 hours of the study and in all donors studied. Thus, the silicone gel composition shows a profile of non-transdermal permeation under the test conditions (lower than 180 ng/cm²), and it can be extrapolated that the product is safe and effective for topical use in human skin. It is concluded that Juglone is not absorbed in the skin, not penetrated but yes permeated at the epidermis depth range.

### - Silicone gel compositions of composition A and composition B were prepared -

### Example 1: Silicone Gel Composition "A"

Components of silicone gel composition are included below:

| Silicone Gel Composition "A" | % (w/w) |
|---|---|
| Walnut (*Juglans Regia L.*) extract | 10.00 |
| Juglone 0.2% w/w | **0.02** |
| Cyclomethicone dimethicone crosspolymer (ST-Elastomer-10 ^{®}) | 68.00 |
| Cyclopentasiloxane (ST-Cyclomethicone 5 NF^{®}) | 18.00 |
| Alkylmethyl silicone polyether copolymer (Dowsil 5200 Formulation Aid^{®}) | 4.00 |

### Example 2: Silicone Gel Composition "B"

In this example, the same components and percentages of example 1 were used except that Juglone was present in a higher amount:

| Silicone Gel Composition "B" | % (w/w) |
|---|---|
| Walnut (*Juglans Regia L.*) extract | 10.00 |
| Juglone 0.4% w/w | **0.04** |
| Cyclomethicone dimethicone crosspolymer (ST-Elastomer-10 ^{®}") | 68.00 |
| Cyclopentasiloxane ("ST-Cyclomethicone 5 NF^{®}") | 18.00 |
| Alkylmethyl silicone polyether copolymer ("Dowsil 5200 Formulation Aid^{®}") | 4.00 |

### Example 3: Preparation of silicone gel composition (A or B).

Ingredients at the in the order shown in the scheme below were added In a central, anchor-stirred reactor, vacuum. The process was performed at room temperature between 15-25°C. Once completed the blend, the product was packaged in 10 ml recipients.

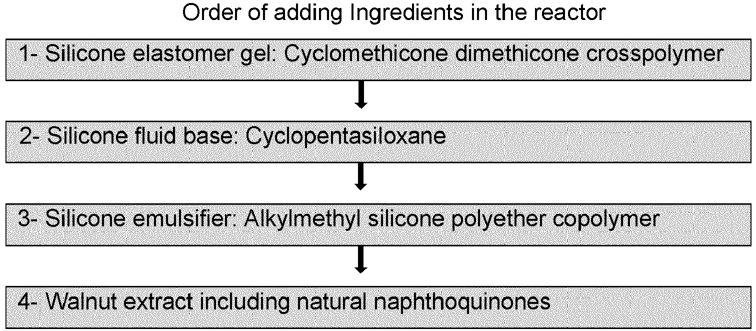

The same methodology was followed for the preparation of silicone gel compositions A and B described in Examples 1 and 2.

## Claims

1. Silicone gel composition comprising walnut extract, **characterized in that** the composition comprises:
- a silicone mixture consisting of: a silicone elastomer gel, a silicone fluid base, and a silicone emulsifier; and
- a walnut extract including natural naphthoquinones ingredient,
wherein the silicone elastomer gel is in an amount between 60 and 75%, the silicone fluid base is in an amount between 12 and 25%, the silicone emulsifier is in an amount between 2 and 6%, and the walnut extract is in an amount between 5 and15%, and
wherein the percentages are expressed by weight with respect to the weight of composition, provided that the sum of all components does not exceed 100%.

2. Silicone gel composition according to claim 1, wherein the silicone elastomer gel includes decamethylcyclopentasiloxane (D5) in a network backbone of polydimethylsiloxane (PDMS), the silicone fluid base includes a cyclopentasiloxane compound, and the silicone emulsifier includes an alkylmethyl silicone polyether copolymer compound.

3. Silicone gel composition according to any one of claims 1-2, wherein the silicone elastomer gel includes decamethylcyclopentasiloxane (D5) in an amount of about 85% by weight and a crosslinked polymer of average molecular weight higher than 5,000 in an amount of about 12% by weight.

4. Silicone gel composition according to any one of claims 1-2, wherein the silicone fluid base includes decamethylcyclopentasiloxane (D5) in an amount higher than about 95% by weight.

5. Silicone gel composition according to any one of claims 1-2, wherein the silicone emulsifier includes alkylmethyl silicone polyether copolymer.

6. Silicone gel composition according to any one of previous claims, wherein the silicone elastomer gel is in an amount between 67 and 69% by weight with respect to the weight of composition.

7. Silicone gel composition according to any one of previous claims, wherein the silicone fluid base is in an amount between 17 and 19% by weight with respect to the weight of composition.

8. Silicone gel composition according to any one of previous claims, wherein the silicone emulsifier is in an amount between 3 and 5% by weight with respect to the weight of composition.

9. Silicone gel composition according to any one of previous claims, wherein the walnut extract is in an amount between 8 and 12% by weight with respect to the weight of composition.

10. Silicone gel composition according to any one of previous claims, wherein walnut extract includes Juglone as natural naphthoquinone ingredient in an amount between 0.1 and 0.5% by weight with respect to the weight of walnut extract.

11. Silicone gel composition comprising walnut extract as defined in claims 1 to 10 for topical use in the treatment of herpes virus.

12. Silicone gel composition for topical use according to claim 11, wherein the use comprises a dosage regime, administered at 3-5-hourly intervals, of 125 to 175 mg up to a total amount of 500 to 600 mg.

13. Silicone gel composition for topical use according to claims 11-12, wherein the dosage regime administered at 4-hourly intervals, is of 125 to 175 mg up to a total of 500 mg.

14. Silicone gel composition for topical use according to claims 11-12, wherein the dosage regime administered at 4-hourly intervals, is of 150 mg up to a total of 600 mg.

15. Silicone gel composition for topical use according to any one of claims 11-14, wherein the treatment is in a target epidermal depth range from about 30 µm to about 120 µm.

## Patentansprüche

1. Silikongelzusammensetzung umfassend Walnussextrakt, **dadurch gekennzeichnet, dass** die Zusammensetzung umfasst:
- eine Silikonmischung bestehend aus: einem Silikonelastomergel, einer Silikonfluidbasis, und einem Silikonemulgator; und
- ein Walnussextrakt, der natürlichen Naphthochinone-Bestandteil enthält,
wobei das Silikonelastomergel in einer Menge zwischen 60 und 75 %, die Silikonfluidbasis in einer Menge zwischen 12 und 25 %, der Silikonemulgator in einer Menge zwischen 2 und 6 % und der Walnussextrakt in einer Menge zwischen 5 und 15 % enthalten ist, und
wobei die Prozentsätze in Gewicht, bezogen auf das Gewicht der Zusammensetzung, ausgedrückt sind, vorausgesetzt, dass die Summe aller Bestandteile 100 % nicht überschreitet.

2. Silikongelzusammensetzung nach Anspruch 1, wobei das Silikonelastomergel Decamethylcyclopentasiloxan (D5) in einem Netzwerkgerüst von Polydimethylsiloxan (PDMS) enthält, die Silikonfluidbasis eine Cyclopentasiloxan-Verbindung enthält, und der Silikonemulgator eine Alkylmethylsilikonpolyether-Copolymerverbindung enthält.

3. Silikongelzusammensetzung nach einem der Ansprüche 1 - 2, wobei das Silikongel Decamethylcyclopentasiloxan (D5) in einer Menge von etwa 85 Gew.-% und ein vernetztes Polymer mit einem durchschnittlichen Molekulargewicht von mehr als 5.000 in einer Menge von etwa 12 Gew.-% enthält.

4. Silikongelzusammensetzung nach einem der Ansprüche 1 - 2, wobei die Silikonfluidbasis Decamethylcyclopentasiloxan (D5) in einer Menge von mehr als etwa 95 Gew.-% enthält.

5. Silikongelzusammensetzung nach einem der Ansprüche 1 - 2, wobei der Silikonemulgator Alkylmethylsilikonpolyether-Copolymer enthält.

6. Silikongelzusammensetzung nach einem der vorhergehenden Ansprüche, wobei das Silikonelastomergel in einer Menge zwischen 67 und 69 Gew.-%, bezogen auf das Gewicht der Zusammensetzung, vorliegt.

7. Silikongelzusammensetzung nach einem der vorhergehenden Ansprüche, wobei die Silikonfluidbasis in einer Menge zwischen 17 und 19 Gew.-%, bezogen auf das Gewicht der Zusammensetzung, vorliegt.

8. Silikongelzusammensetzung nach einem der vorhergehenden Ansprüche, wobei der Silikonemulgator in einer Menge zwischen 3 und 5 Gew.-%, bezogen auf das Gewicht der Zusammensetzung, vorliegt.

9. Silikongelzusammensetzung nach einem der vorhergehenden Ansprüche, wobei der Walnussextrakt in einer Menge zwischen 8 und 12 Gew.-%, bezogen auf das Gewicht der Zusammensetzung, vorliegt.

10. Silikongelzusammensetzung nach einem der vorhergehenden Ansprüche, wobei der Walnussextrakt Juglon als natürlichen Naphthochinon-Bestandteil in einer Menge zwischen 0,1 und 0,5 Gew.-%, bezogen auf das Gewicht des Walnussextrakts, enthält.

11. Silikongelzusammensetzung, umfassend Walnussextrakt gemäß den Ansprüchen 1 bis 10 zur topischen Anwendung bei der Behandlung von Herpesviren.

12. Silikongelzusammensetzung zur topischen Anwendung nach Anspruch 11, wobei die Anwendung ein Dosierungsschema, verabreicht in Abständen von 3 - 5 Stunden, von 125 bis 175 mg bis zu einer Gesamtmenge von 500 bis 600 mg umfasst.

13. Silikongelzusammensetzung zur topischen Anwendung nach den Ansprüchen 11 - 12, wobei das Dosierungsschema, verabreicht in Abständen von 4 Stunden, 125 bis 175 mg bis zu einer Gesamtmenge von 500 mg beträgt.

14. Silikongelzusammensetzung zur topischen Anwendung nach den Ansprüchen 11 - 12, wobei das Dosierungsschema, verabreicht in Abständen von 4 Stunden, 150 mg bis zu einer Gesamtmenge von 600 mg beträgt.

15. Silikongelzusammensetzung zur topischen Anwendung nach einem der Ansprüche 11 - 14, wobei die Behandlung in einem Zielbereich von etwa 30 µm bis etwa 120 µm epidermaler Tiefe liegt.

## Revendications

1. Composition de gel de silicone comprenant de l'extrait de noix, **caractérisée par le fait que** la composition comprend :
- un mélange de silicone composé d'un gel élastomère de silicone, d'une base fluide de silicone et d'un émulsifiant de silicone ; et
- un extrait de noix contenant un ingrédient naturel, la naphtoquinone,
dans laquelle le gel élastomère de silicone représente une quantité comprise entre 60 et 75 %, la base fluide de silicone représente une quantité comprise entre 12 et 25 %, l'émulsifiant de silicone représente une quantité comprise entre 2 et 6 %, et l'extrait de noix représente une quantité comprise entre 5 et 15 %, et
dans laquelle les pourcentages sont exprimés en poids par rapport au poids de la composition, à condition que la somme de tous les composants n'excède pas 100 %.

2. Composition de gel de silicone selon la revendication 1, dans laquelle le gel d'élastomère de silicone comprend du décaméthylcyclopentasiloxane (D5) dans une ossature de réseau de polydiméthylsiloxane (PDMS), la base fluide de silicone comprend un composé de cyclopentasiloxane, et l'émulsifiant de silicone comprend un composé copolymère alkylméthyl de silicone polyéther.

3. Composition de gel de silicone selon l'une quelconque des revendications 1-2, dans laquelle le gel élastomère de silicone comprend du décaméthylcyclopentasiloxane (D5) dans une proportion d'environ 85 % en poids et un polymère réticulé de poids moléculaire moyen supérieur à 5 000 dans une quantité d'environ 12 % en poids.

4. Composition de gel de silicone selon l'une quelconque des revendications 1-2, dans laquelle la base fluide de silicone comprend du décaméthylcyclopentasiloxane (D5) dans une quantité supérieure à environ 95 % en poids.

5. Composition de gel de silicone selon l'une quelconque des revendications 1-2, dans laquelle l'émulsifiant de silicone comprend un copolymère alkylméthyl de silicone polyéther

6. Composition de gel de silicone selon l'une quelconque des revendications précédentes, dans laquelle le gel élastomère de silicone est dans une quantité comprise entre 67 et 69 % en poids par rapport au poids de la composition.

7. Composition de gel de silicone selon l'une quelconque des revendications précédentes, dans laquelle la base de fluide de silicone est dans une quantité comprise entre 17 et 19 % en poids par rapport au poids de la composition.

8. Composition de gel de silicone selon l'une quelconque des revendications précédentes, dans laquelle l'émulsifiant de silicone est dans une quantité comprise entre 3 et 5 % en poids par rapport au poids de la composition.

9. Composition de gel de silicone selon l'une quelconque des revendications précédentes, dans laquelle l'extrait de noix est dans une quantité comprise entre 8 et 12 % en poids par rapport au poids de la composition.

10. Composition de gel de silicone selon l'une quelconque des revendications précédentes, dans laquelle l'extrait de noix comprend du Juglon comme ingrédient naturel de naphtoquinone dans une quantité comprise entre 0,1 et 0,5 % en poids par rapport au poids de la composition.

11. Composition de gel de silicone comprenant de l'extrait de noix tel que défini dans les revendications 1 à 10 pour une utilisation topique dans le traitement du virus de l'herpès.

12. Composition de gel de silicone à usage topique selon la revendication 11, dans laquelle l'utilisation comprend un régime posologique, administré à des intervalles de 3 à 5 heures, de 125 à 175 mg jusqu'à une quantité totale de 500 à 600 mg.

13. Composition de gel de silicone à usage topique selon les revendications 11-12, dans laquelle le régime posologique administré à des intervalles de 4 heures, est de 125 à 175 mg jusqu'à un total de 500 mg.

14. Composition de gel de silicone à usage topique selon les revendications 11-12, dans laquelle le régime posologique administré à des intervalles de 4 heures, est de 150 mg jusqu'à un total de 600 mg.

15. Composition de gel de silicone à usage topique selon l'une quelconque des revendications 11 à 14, dans laquelle le traitement s'effectue à une profondeur épidermique cible d'environ 30 µm à environ 120 µm.
